# EUROPÄISCHE PATENTANMELDUNG

(11) **EP 4 151 200 A1**
(43) Veröffentlichungstag der Anmeldung: **22.03.2023**
(21) Anmeldenummer: 22187388.8
(22) Anmeldetag: 28.07.2022
(51) Int. Cl.: A61K 8/34, A61K 8/35, A61K 8/37, A61K 8/40, A61K 8/49, A61K 8/92, A61Q 5/00, A61Q 17/04, A61Q 19/00

(54) **KOSMETISCHES MITTEL MIT UV-SCHUTZ**

(30) Priorität: 17.09.2021 DE 102021210362
(71) Anmelder: Henkel AG & Co. KGaA, 40589 Düsseldorf (DE)
(72) Erfinder: Delowsky, Jens, 22844 Norderstedt (DE); Kerl, Sylvia, 25474 Bönningstedt (DE); Glasing, Joe, 22763 Hamburg (DE)

(57) **Zusammenfassung**

Die vorliegende Erfindung betrifft kosmetisches Sonnenschutz- und pflegemittel, die in einer alkoholischen Basis UVA- und UVB-Filter sowie spezifische Öle enthalten.

## Beschreibung

Die Erfindung betrifft kosmetische Sonnenschutzmittel, die in einer alkoholischen Basis eine spezifische Mischung aus UV-Filtern und Emollients enthalten, sowie die Verwendung der Mittel für den Schutz von Haut und Haaren vor UV-Strahlen.

Es ist bekannt, dass menschliche Haut oder Haare durch Sonneneinwirkung massiv geschädigt werden können. Ursächlich dafür ist hauptsächlich ein bestimmter Anteil des Sonnenlichts, welcher nicht von der Ozonschicht und dem Luftsauerstoff absorbiert wird und als Strahlung im UVA-Bereich (Wellenlänge zwischen 400 bis 320 nm) und UVB-Bereich (Wellenlänge von 320 bis 285 nm) auftritt. Als schädliche Folgen länger andauernder oder häufiger Sonnenexposition können Sonnenbrände, Erythreme, Hautnekrosen, Faltenbildung (vorzeitige Hautalterung) und/oder Strukturveränderungen von Haaren auftreten, die zum Teil irreversibel sind und im schlimmsten Fall zu malignen Veränderungen der Haut führen können.

Der Schutz des menschlichen Körpers vor UV-Strahlen ist demzufolge ein wichtiges Ziel bei der Bereitstellung kosmetischer Mittel, wobei dem Schutz des menschlichen Kopfes eine besondere Bedeutung zukommt, denn dieser kann nicht immer ausreichend mittels geeigneter Bekleidung vor Sonneneinwirkung geschützt werden.

Für den UV-Schutz der Haut ist eine Vielzahl unterschiedlichster Produkte bekannt. Die allermeisten basieren auf einer Emulsionsgrundlage und eignen sich nicht bzw. nur sehr eingeschränkt für die gleichzeitige Behandlung der Haare oder der Kopfhaut, denn sie verkleben die Haare und verleihen ihnen ein ungepflegtes, fettiges Erscheinungsbild.

Haarsprays mit UV-Schutzwirkung wiederum weisen oftmals keinen zufriedenstellenden Haarpflegeeffekt auf und können die Kopfhaut nur sehr unzureichend vor UV-Strahlen schützen.

Benötigt werden deshalb kosmetische Haut- und Haarpflegeprodukte mit UV-Schutzwirkung, die im gesamten Kopfbereich angewendet werden können.

Aufgabe der vorliegenden Erfindung war es kosmetische Produkte mit Lichtschutzfaktor (SPF) zur Verfügung zu stellen, welche die Haut und die Haare vor UVA- und UVB-Strahlen schützen und gleichzeitig pflegen. Optimalerweise sollten Produkte zur Verfügung gestellt werden, mit denen sich in einem einzigen Behandlungsschritt schnell und unkompliziert alle Bereiche des menschlichen Kopfes pflegen und gleichzeitig vor Sonnenstrahlung schützen lassen.

Ein weiteres Ziel war die Bereitstellung zuvor genannter kosmetischer Pflegeprodukte mit UV-Schutzwirkung, bei denen auf den Einsatz von Silikonen verzichtet werden kann.

Diese Aufgaben wurden erfindungsgemäß durch das Bereitstellen eines kosmetischen Mittels gemäß Anspruch 1 gelöst.

Ein erster Gegenstand der vorliegenden Erfindung ist demnach ein kosmetisches Sonnenschutzmittel, das
a) 10 - 30 Gew.-% einer UV-Filtermischung, umfassend mindestens einen UVA- und mindestens einen UVB-Filter,
b) 1 - 10 Gew.-% mindestens eines Öls mit einem Spreitwert > 500 mm²/10 min. und
c) 50 - 80 Gew.-% mindestens eines C1-C6-Alkohols
enthält, wobei sich die Mengenangaben auf das Gesamtgewicht des Sonnenschutzmittels beziehen.

Die erfindungsgemäßen Mittel gewährleisten einen Schutz vor UVA- und UVB-Strahlen. Gleichzeitig weisen damit behandelte Kopfpartien, insbesondere die Haare, ein gepflegtes Erscheinungsbild auf, denn die Mittel hinterlassen keinen Fettfilm, wodurch auch keine Beschwerung der Haare und/oder ein Fettglanz der Haut auftritt.

Die Verwendung von Silikonen zur Erzielung der Pflegewirkung ist nicht erforderlich.

Ein weiterer besonderer Vorteil der erfindungsgemäßen Mittel besteht darin, dass sie transparent und lagerstabil sind, selbst wenn sie als niederviskose, versprühbare Formulierung konfektioniert werden. Eine flüssige oder fließfähige Darreichungsform der erfindungsgemäßen Mittel bietet den besonderen Vorteil, dass sowohl bestimmte Kopfpartien wie ein Scheitel oder kahle Stellen am Kopf gezielt mit einem erfindungsgemäßen Mittel - beispielsweise in Form eines Serums - behandelt werden können, als auch dass eine Sprühapplikation möglich ist, bei der einfach und zügig durch mehrfache Betätigung eines Sprühkopfes alle Bereiche des Kopfes (Haare, Scheitel, kahle Stellen, Gesicht, Hals) erreicht werden können.

Um den gewünschten Schutz vor UV-Strahlen zu erreichen, enthalten die erfindungsgemäßen Sonnenschutzmittel eine spezifische UV-Filtermischung, umfassend mindestens einen UVA- und mindestens einen UVB-Filter in einem Gewichtsanteil von 10 - 30 Gew.-% am Gesamtgewicht des Mittels.

Besonders bevorzugt sind Einsatzmengen der UV-Filtermischung von 12,5 - 30 Gew.-%, ganz besonders bevorzugt 15 - 30 Gew.-% und insbesondere 17,5 - 27,5 Gew.-%.

Bei der Auswahl geeigneter UVA- und UVB-Filtertypen sowie deren Einsatzmengen sind zunächst die gesetzlichen Bestimmungen in den für die Vermarktung der Produkte vorgesehenen Ländern oder Wirtschaftsräume zu beachten.

Daneben sind auch formulierungstechnische Aspekte zu berücksichtigen, denn die gängigsten UV-Filtersubstanzen weisen unterschiedliche Aggregatzustände und Löslichkeiten auf und erfordern bei ihrer Kombination und Einarbeitung in kosmetische Grundlagen meist aufwendige Stabilisierungsschritte.

Geeignete UVA-Filter können ausgewählt sein aus chemischen und mineralischen UVA-Filtern sowie Mischungen davon.

Bevorzugte chemische UVA-Filter im Sinne der vorliegenden Erfindung sind beispielsweise die unter den Handels- und/oder INCI-Bezeichnungen erhältlichen Verbindungen: Avobenzone (Butyl Methoxydibenzoylmethane), Bis-Mexoryl^{®} SX (Terephthalydene Dicamphor Sulfonic Acid), Tinosorb^{®} S oder Bemotrizinol (Bis-Ethylhexyloxyphenol Methoxyphenyl Triazine), Uvinul^{®}-A-plus (Diethylamino Hydroxybenzoyl Hexyl Benzoate) oder Mischungen davon.

Besonders bevorzugt sind Avobenzone, Bemotrizinol oder Mischungen davon und insbesondere bevorzugt ist Avobenzone.

Bevorzugte mineralische UVA-Filter im Sinne der vorliegenden Erfindung sind beispielsweise die Oxide des Titans, Zinks, Eisens, Zirkoniums, Siliciums, Mangans, Aluminiums, Cers und Mischungen davon, sowie Abwandlungen, bei denen Oxide die aktiven Agentien sind. Besonders bevorzugt sind Titandioxid, Zinkoxid und Mischungen davon.

Der mindestens eine UVA-Filter kann in den erfindungsgemäßen Mitteln in einem Gewichtsanteil von 1 - 10 Gew.-% am Gesamtgewicht des kosmetischen Mittels eingesetzt werden. Bevorzugt sind Mengen von 2 - 9 Gew.-%, besonders bevorzugt von 2,5 - 8 Gew.-% und insbesondere von 3 - 7,5 Gew.-%.

In einer besonders bevorzugten Ausführungsform enthalten die erfindungsgemäßen Mittel als UVA-Filter Bemotrizinol, Avobenzone oder Mischungen davon in den zuvor genannten Mengen. Innerhalb dieser Ausführungsform ist es besonders bevorzugt, wenn die erfindungsgemäßen Mittel Avobenzone in den zuvor genannten Mengen enthalten.

Geeignete UVB-Filter können beispielsweise ausgewählt sein aus
- 3-Benzylidencampher und dessen Derivaten wie 3-(4-Methylbenzyliden)campher
- 4-Aminobenzoesäurederivaten wie 4-(Dimethylamino)-benzoesäure(2-ethylhexyl)ester, 4-(Dimethylamino)-benzoesäuremethylester
- Ester der Zimtsäure wie 4-Methoxyzimtsäure(2-ethylhexyl)ester, 4-Methoxyzimtsäureisopentylester
- Ester der Salicylsäure wie Salicylsäure(2-ethylhexyl)ester (Octisalate), Salicylsäure(4-isopropylbenzyl)ester, Salicylsäurehomomenthylester, 2-Hydroxybenzoesäure-3,3,5-trimethylcyclohexylester (Homosalate)
- Acrylsäurederivate wie 2-Ethylhexyl-2-cyano-3,3-diphenylacrylat (Octocrylene)
- Derivate des Benzophenons wie 2-Hydroxy-4-methoxybenzophenon, 2-Hydroxy-4-methoxy-4'-methylbenzophenon, 2,2'-Dihydroxy-4-methoxy-benzophenon
- Ester der Benzalmalonsäure wie 4-Methoxybenzalmalonsäure-di-(2-ethylhexyl)ester,
- 2,4,6-Trianilino-(p-carbo-2'-ethyl-1'-hexyloxy)-1,3,5-triazin
- Sulfonsäurederivate wie 2-Phenylbenzimidazol-5-sulfonsäure und deren Natrium-, Kalium- oder Triethanolaminsalze (Ensulizol)
- Sulfonsäurederivate von Benzophenonen wie 2-Hydroxy-4-methoxybenzophenon-5-sulfonsäure
- Derivate des 3-Benzylidencamphers wie 4-(2-Oxo-3-bornylidenmethyl)benzolsulfonsäure, 2-Methyl-5-(2-oxo-3- bornylidenmethyl)benzolsulfonsäure sowie Salze davon.

Der mindestens eine UVB-Filter kann in den erfindungsgemäßen Mitteln in einem Gewichtsanteil von 10 - 30 Gew.-% am Gesamtgewicht des kosmetischen Mittels eingesetzt werden. Bevorzugt sind Mengen von 12 - 30 Gew.-%, besonders bevorzugt von 12,5 - 27,5 Gew.-% und insbesondere von 12,5 - 25 Gew.-%.

In einer besonders bevorzugten Ausführungsform enthalten die erfindungsgemäßen Mittel als UVB-Filter Octisalate (Ethylhexyl Salicylate), Homosalate, Octocrylene, Ensulizol (Phenylbenzimidazole sulfonic acid) oder Mischungen davon in den zuvor genannten Mengen.

Innerhalb dieser Ausführungsform ist es besonders bevorzugt, wenn die erfindungsgemäßen Mittel Homosalate, Octocrylene oder Mischungen davon in den zuvor genannten Mengen enthalten. Innerhalb dieser Ausführungsform ist es weiterhin besonders bevorzugt, wenn die erfindungsgemäßen Mittel Ethylhexyl Salicylate, Octocrylene, Phenylbenzimidazole sulfonic acid oder Mischungen davon in den zuvor genannten Mengen enthalten.

Ganz besonders bevorzugte UV-Filtermischungen a) im Sinne der vorliegenden Erfindung enthalten - bezogen auf das Gesamtgewicht der erfindungsgemäßen Sonnenschutzmittel -
(i) 1 - 10 Gew.-% Avobenzone,
(ii) 5 - 15 Gew.-% Homosalate und
(iii) 5 - 15 Gew.-% Octocrylene.

Weiterhin ganz besonders bevorzugte UV-Filtermischungen a) im Sinne der vorliegenden Erfindung enthalten - bezogen auf das Gesamtgewicht der erfindungsgemäßen Sonnenschutzmittel -
(i) 1 - 10 Gew.-% Ethylhexyl Salicylate,
(ii) 5 - 15 Gew.-% Octocylene und
(iii) 1 - 10 Gew.-% Phenylbenzimidazole sulfonic acid.

Es wurde gefunden, dass die erfindungsgemäße UV-Filtermischung besonders gut gelöst und in einer alkoholischen Basis stabilisiert werden kann, wenn in Wasser praktisch unlösliche UV-Filterwirkstoffe zunächst in bestimmten Emollientien vorgelöst und anschließend in die alkoholische Basis eingearbeitet werden.

"In Wasser unlöslich", "wasserunlöslich" oder "praktisch in Wasser unlöslich" bedeutet, dass der oder die UV-Filter bei 25°C in Wasser zu weniger als 1 g/l gelöst werden.

Als besonders geeignet haben sich dabei Emollientien mit einem Spreitwert > 500 mm²/10 min. erwiesen, denn sie gewährleisten nicht nur die sehr gute Auflösung erfindungsgemäß geeigneter UV-Filterwirkstoffe, sondern sie lassen sich zudem besonders gut und ohne besonderen energetischen Aufwand in die alkoholische Basis der erfindungsgemäßen Mittel einarbeiten und hinterlassen auf der Anwendungsoberfläche außerdem einen ausgezeichneten Pflegeeffekt. Weiterhin vorteilhaft ist, dass durch die Verwendung spezifischer Emollientien stabile, fließfähige, einfach handhabbare, transparente Zusammensetzungen bereitgestellt werden können.

Die erfindungsgemäßen Sonnenschutzmittel enthalten demzufolge mindestens ein Öl mit einem Spreitwert > 500 mm²/10 min. in einem Gewichtsanteil von 1 - 10 Gew.-% am Gesamtgewicht des Sonnenschutzmittels.

Bevorzugt sind Mengen von 2,5 - 10 Gew.-%, besonders bevorzugt 3 bis 9 Gew.-% und insbesondere 4 bis 8 Gew.-%.

Geeignete Öle mit einem Spreitwert > 500 mm²/10 min. können ausgewählt sein aus Verbindungen mit den INCI-Bezeichnungen Isopropyl palmitate, Isopropyl myristate, Coco caprylate/caprate, Isopropyl isostearate, Butylene Glycol dicaprylate/dicaprate, Di-(2-ethylhexyl) carbonate, Dicaprylyl carbonate, Isopropyl stearate, Ethylhexyl isononanoate, Ethylhexyl cocoate, Dibutyl adipate, Isodecyl neopentanoate, Isodecane, Isoundecane, Isododecane, Isotridecane, Isotertadecane, Isopentadecane, Isohexadecane, Dicaprylyl Ether, Hexyl Laurate, Heptyl Undecylate sowie aus natürlichen Ölen wie beispielsweise Squalan, Babassuöl, Kokosöl oder aus beliebigen Mischungen der zuvor genannten Öle.

Bevorzugt im Sinne der vorliegenden Erfindung sind schnell (hoch-)spreitende Öle mit einem Spreitwert > 800 mm²/10 min. oder Mischungen von Ölen mit einem Spreitwert von 500 - 800 mm²/10 min. und Ölen mit einem Spreitwert von > 800 mm²/10 min.

Derartige Öle oder Mischungen von Ölen weisen den Vorteil auf, dass sie sich besonders gut und rasch auf der Anwendungsoberfläche verteilen, wodurch auch die UV-Filterwirkstoffe gut und gleichmäßig verteilt werden. Zudem hinterlassen sie ein angenehmes, glattes, kaum fettendes Gefühl auf der Haut und/oder den Haaren.

Besonders bevorzugt enthalten die erfindungsgemäßen Sonnenschutzmittel das oder die Öle:
- Dicaprylyl carbonate,
- Dicaprylyl carbonate und Kokosöl,
- Isododecane,
- Isododecane und Kokosöl,
- Isopropylmyristate,
- Ethylhexyl isononanoate,
- Isopropylmyristate und Kokosöl,
- Ethylhexyl isononanoate und Kokosöl,
- Isopropylmyristate, Ethylhexyl isononanoate und Kokosöl,
- Caprylic/Capric Triglyceride.

In einer besonders bevorzugten Ausführungsform enthalten erfindungsgemäße Sonnenschutzmittel Dicaprylyl carbonate in einem Gewichtsanteil von 2 - 7,5 Gew.-% am Gesamtgewicht des Mittels.

In einer weiteren bevorzugten Ausführungsform enthalten erfindungsgemäße Sonnenschutzmittel Dicaprylyl carbonate in einem Gewichtsanteil von 2 - 7,5 Gew.-% und 0,05 - 1 Gew.-% Kokosöl am Gesamtgewicht des Mittels.

In einer weiteren besonders bevorzugten Ausführungsform enthalten erfindungsgemäße Sonnenschutzmittel Isopropylmyristat, Ethylhexyl isononanoate oder Mischungen davon in einem Gewichtsanteil von 2 - 9 Gew.-% am Gesamtgewicht des Mittels.

In einer weiteren besonders bevorzugten Ausführungsform enthalten erfindungsgemäße Sonnenschutzmittel Isopropylmyristat, Ethylhexyl isononanoate oder Mischungen davon in einem Gewichtsanteil von 2 - 9 Gew.-% und Kokosöl in einem Gewichtsanteil von 0,05 - 1 Gew.-% am Gesamtgewicht des kosmetischen Mittels.

In einer weiteren bevorzugten Ausführungsform sind die erfindungsgemäßen Sonnenschutzmittel frei von Silikonen.

Im Zusammenhang der vorliegenden Erfindung bedeutet "frei von Silikonen", dass das Sonnenschutzmittel frei von Organosiliziumverbindungen, insbesondere frei von Silikonen, Trisiloxanen und Silikonölen ist.

Als dritte wesentliche Komponente enthalten die erfindungsgemäßen Sonnenschutzmittel 50 - 80 Gew.-% mindestens eines C1-C6-Alkohols, bezogen auf das Gesamtgewicht des Mittels. Bevorzugt sind Mengen von 55 - 80 Gew.-%, besonders bevorzugt 60 - 80 Gew.-% und insbesondere bevorzugt 65 - 80 Gew.-%.

Die UV-Filtermischung sowie die Emollientien können in einer alkoholischen Basis besonders gut gelöst oder dispergiert werden und gewährleisten die Bereitstellung stabiler, transparenter Pflegezusammensetzungen mit UV-Schutz und leichter Textur, die vorzugsweise niedrigviskos formuliert werden und sich dadurch hervorragend für die Sprühapplikation oder für die Applikation aus einem Spender mit Pipette eignen. Darüber hinaus verdunsten die Alkohole auf der Anwendungsoberfläche rasch und hinterlassen darauf neben einem angenehmen, erfrischendkühlenden sensorischen Effekt eine hochwirksame UV-Schutz-Pflegewirkstoffmischung ohne die Haare auch nach mehrfacher Anwendung zu beschweren.

Unter "niedrigviskos" wird vorzugsweise eine Viskosität im Bereich von 500 bis 5000 mPas, mehr bevorzugt 500 bis 3000 mPas verstanden. Die Viskositätsmessung erfolgte mit einem Rotationsviskosimeter, Modell VT 550 der Firma Haake, bei einer Temperatur von 20°C und 8 UpM.

Unter "Transparenz" wird im Sinne der vorliegenden Erfindung verstanden, dass die erfindungsgemäßen Sonnenschutzmittel Licht in dem für Menschen gewöhnlich sichtbaren Bereich nicht streuen.

Vorzugsweise ist die Basis der erfindungsgemäßen Sonnenschutzmittel wasserfrei, worunter verstanden wird, dass die Mittel kein zugesetztes Wasser enthalten. Wasser als Nebenbestandteil von Handelsprodukten oder Wirkstoffmischungen ist dabei ausgenommen, jedoch beträgt auch in diesem Fall der Gesamtgehalt an Wasser (bezogen auf das Gesamtgewicht des Sonnenschutzmittels) vorzugsweise maximal 1 Gew.-%, besonders bevorzugt maximal 0,75 Gew.-% und insbesondere bevorzugt maximal 0,5 Gew.-%.

Geeignete C1-C6-Alkohole im Sinne der vorliegenden Erfindung sind vorzugsweise ausgewählt aus Ethanol, Propanol, Isopropanol, Propylenglycol, Butylenglycol, Pentylenglycol, Dipropylenglycol, Glycerin, Diglycerin oder Mischungen davon.

Besonders bevorzugt ist Ethanol oder Mischungen von Ethanol als Hauptbestandteil der alkoholischen Basis mit geringen Mengen an Glykolen und/oder Glycerin.

In einer bevorzugten Ausführungsform umfassen erfindungsgemäße Sonnenschutzmittel 55 - 80 Gew.-% Ethanol (bezogen auf das Gesamtgewicht des Mittels). Besonders bevorzugt sind 60 - 80 Gew.-% und insbesondere bevorzugt 65 - 75 Gew.-% Ethanol.

In einer weiteren besonders bevorzugten Ausführungsform enthalten erfindungsgemäße Sonnenschutzmittel 55 - 80 Gew.-% Ethanol sowie 0 - 10 Gew.-% 1,2-Propylenglycol, Glycerin oder Mischungen davon (bezogen auf das Gesamtgewicht des Mittels).

Neben den wesentlichen Inhaltsstoffen können die erfindungsgemäßen Sonnenschutzmittel weitere Inhaltsstoffe umfassen, die ihnen vorteilhafte Eigenschaften verleihen.

So wurde gefunden, dass ein geringer Anteil an Filmbildnern die gleichmäßige Verteilung sowie die Haftung von UV-Schutzwirkstoffen und Emollientien auf der Anwendungsoberfläche unterstützen oder verbessern kann.

Unter Filmbildnern sind vor allem synthetische oder natürliche Polymere zu verstehen, welche beim Trocknen einen kontinuierlichen Film auf der Haut, dem Haar oder den Nägeln hinterlassen.

In einer bevorzugten Ausführungsform enthalten erfindungsgemäße Sonnenschutzmittel zusätzlich mindestens einen Filmbildner in einem Gewichtsanteil von 0,1 - 1,5 Gew.-% am Gesamtgewicht des Mittels. Bevorzugt sind Mengen von 0,2 - 1,5 Gew.-%, besonders bevorzugt 0,25 - 1,25 Gew.-% und insbesondere 0,25 - 1 Gew.-%.

Geeignete Filmbildner im Sinne der vorliegenden Erfindung können beispielsweise ausgewählt sein aus Polyestern, Polyamiden, Homo- und/oder Copolymeren von Acrylsäure- und/oder Methacrylsäure sowie deren Derivaten, PVP und Derivaten davon, Wachsen, Proteinen, Chitosan und Derivaten davon, Galactomannanen, Cellulosen, Stärken oder Mischungen dieser Filmbildner.

Spezifische Beispiele erfindungsgemäß geeigneter Filmbildner sind:
Abies Balsamea (Balsam Canada) Resin, Acetylenediurea/Formaldehyde/Tosylamide Crosspolymer, Acrylamide/Ammonium Acrylate Copolymer, Acrylamides Copolymer, Acrylamides/DMAPA Acrylates/Methoxy PEG Methacrylate Copolymer, Acrylamide/Sodium Acrylate Copolymer, Acrylamidopropyltrimonium Chloride/Acrylamide Copolymer, Acrylamidopropyltrimonium Chloride/Acrylates Copolymer, Acrylates/Acetoacetoxyethyl Methacrylate Copolymer, Acrylates/Acrylamide Copolymer, Acrylates/Ammonium Methacrylate Copolymer, Acrylates/Behenyl Methacrylate/Dimethicone Methacrylate Copolymer, Acrylates/t-Butylacrylamide Copolymer, Acrylates Copolymer, Acrylates/Diacetoneacrylamide Copolymer, Acrylates/Dimethicone Copolymer, Acrylates/Dimethicone Methacrylate Copolymer, Acrylates/Dimethiconol Acrylate Copolymer, Acrylates/Dimethylaminoethyl Methacrylate Copolymer, Acrylates/Ethylhexyl Acrylate Copolymer, Acrylates/Ethylhexyl Acrylate/HEMA/Styrene Copolymer, Acrylates/Ethylhexyl Acrylate/Styrene Copolymer, Acrylates/Hydroxyesters Acrylates Copolymer, Acrylates/Lauryl Acrylate/Stearyl Acrylate/Ethylamine Oxide Methacrylate Copolymer, Acrylates/Octylacrylamide Copolymer, Acrylates/Propyl Trimethicone Methacrylate Copolymer, Acrylates/Stearyl Acrylate/Dimethicone Methacrylate Copolymer, Acrylates/Stearyl Acrylate/Ethylamine Oxide Methacrylate Copolymer, Acrylates/TDI/Trimethylolpropane Copolymer, Acrylates/VA Copolymer, Acrylates/VA Crosspolymer, Acrylates/VP Copolymer, Acrylates/VP/Dimethylaminoethyl Methacrylate/Diacetone Acrylamide/Hydroxypropyl Acrylate Copolymer, Acrylic Acid/Acrylonitrogens Copolymer, Adipic Acid/CHDM/MA/Neopentyl Glycol/Trimellitic Anhydride Copolymer, Adipic Acid/Diethylene Glycol/Glycerin Crosspolymer, Adipic Acid/Diethylenetriamine Copolymer, Adipic Acid/Dilinoleic Acid/Hexylene Glycol Copolymer, Adipic Acid/Dimethylaminohydroxypropyl Diethylenetriamine Copolymer, Adipic Acid/Epoxypropyl Diethylenetriamine Copolymer, Adipic Acid/Fumaric Acid/Phthalic Acid/Tricyclodecane Dimethanol Copolymer, Adipic Acid/Isophthalic Acid/Neopentyl Glycol/Trimethylolpropane Copolymer, Adipic Acid/Neopentyl Glycol/Trimellitic Anhydride Copolymer, Adipic Acid/PPG-10 Copolymer, Albumen, Allyl Stearate/VA Copolymer, Aloe Barbadensis Leaf Polysaccharides, Aminoethylacrylate Phosphate/Acrylates Copolymer, Aminoethylpropanediol-Acrylates/Acrylamide Copolymer, Aminoethylpropanediol-AMPD-Acrylates/Diacetoneacrylamide Copolymer, Ammonium Acrylates/Acrylonitrogens Copolymer, Ammonium Acrylates Copolymer, Ammonium Alginate, Ammonium Polyacrylate, Ammonium Styrene/Acrylates Copolymer, Ammonium VA/Acrylates Copolymer, AMPD-Acrylates/Diacetoneacrylamide Copolymer, AMP-Acrylates/Allyl Methacrylate Copolymer, AMP-Acrylates/C1-18 Alkyl Acrylates/C1-8 Alkyl Acrylamide Copolymer, AMP-Acrylates Copolymer, AMP-Acrylates/Diacetoneacrylamide Copolymer, AMP-Acrylates/Dimethylaminoethylmethacrylate Copolymer, Astragalus Gummifer Gum, Avena Sativa (Oat) Kernel Protein, Behenyl Methacrylate/Perfluorooctylethyl Methacrylate Copolymer, Benzoguanamine/Formaldehyde/Melamine Crosspolymer, Benzoic Acid/Phthalic Anhydride/Pentaerythritol/Neopentyl Glycol/Palmitic Acid Copolymer, Bis-Hydrogenated Tallow Amine Dilinoleic Acid/Ethylenediamine Copolymer, Bis-PEG-15 Dimethicone/IPDI Copolymer, Bis-PPG-15 Dimethicone/IPDI Copolymer, Bis-Stearyl Dimethicone, Brassica Campestris/Aleurites Fordi Oil Copolymer, Butadiene/Acrylonitrile Copolymer, 1,4-Butandiol/Succinic Acid/Adipic Acid/HDI Copolymer, Butoxy Chitosan, Butyl Acrylate Crosspolymer, Butyl Acrylate/Ethylhexyl Methacrylate Copolymer, Butyl Acrylate/Hydroxyethyl Methacrylate Copolymer, Butyl Acrylate/Hydroxypropyl Dimethicone Acrylate Copolymer, Butyl Acrylate/Styrene Copolymer, Butylated Polyoxymethylene Urea, Butylated PVP, Butyl Benzoic Acid/Phthalic Anhydride/Trimethylolethane Copolymer, Butylene/Ethylene/Propylene Copolymer, Butyl Ester of Ethylene/MA Copolymer, Butyl Ester of PVM/MA Copolymer, Butylethylpropanediol Dimer Dilinoleate, Butyl Methacrylate/DMAPA Acrylates/Vinylacetamide Crosspolymer, C23-43 Acid Pentaerythritol Tetraester, Calcium Carboxymethyl Cellulose, Calcium Carrageenan, Calcium Potassium Carbomer, Calcium/Sodium PVM/MA Copolymer, C5-6 Alkane/Cycloalkane/Terpene Copolymer, C30-45 Alkyl Dimethicone/Polycyclohexene Oxide Crosspolymer, C1-5 Alkyl Galactomannan, Candelilla Wax Hydrocarbons, Carboxybutyl Chitosan, Carboxymethyl Chitosan, Carboxymethyl Chitosan Succinamide, Carboxymethyl Dextran, Carboxymethyl Hydroxyethylcellulose, Castor Oil/IPDI Copolymer, Cellulose Acetate, Cellulose Acetate Butyrate, Cellulose Acetate Propionate, Cellulose Acetate Propionate Carboxylate, Cellulose Gum, Cetearyl Dimethicone/Vinyl Dimethicone Crosspolymer, Chitosan, Chitosan Adipate, Chitosan Ascorbate, Chitosan Formate, Chitosan Glycolate, Chitosan Lactate, Chitosan PCA, Chitosan Salicylate, Chitosan Succinamide, C5-6 Olefin/C8-10 Naphtha Olefin Copolymer, Collodion, Copaifera Officinalis (Balsam Copaiba) Resin, Copal, Corn Starch/Acrylamide/Sodium Acrylate Copolymer, Corn Starch Modified, C6-14 Perfluoroalkylethyl Acrylate/HEMA Copolymer, DEA-Styrene/Acrylates/DVB Copolymer, Dibutylhexyl IPDI, Didecyltetradecyl IPDI, Diethylene Glycolamine/Epichlorohydrin/Piperazine Copolymer, Diethylhexyl IPDI, Diglycol/CHDM/Isophthalates/SIP Copolymer, Diglycol/Isophthalates/SIP Copolymer, Dihydroxyethyl Tallowamine/IPDI Copolymer, Dilinoleic Acid/Glycol Copolymer, Dilinoleic Acid/Sebacic Acid/Piperazine/Ethylenediamine Copolymer, Dilinoleyl Alcohol/IPDI Copolymer, Dimethicone PEG-8 Polyacrylate, Dimethicone/Vinyltrimethylsiloxysilicate Crosspolymer, Dimethiconol/IPDI Copolymer, Dimethylamine/Ethylenediamine/Epichlorohydrin Copolymer, Dioctyldecyl IPDI, Dioctyldodecyl IPDI, Di-PPG-3 Myristyl Ether Adipate, Divinyldimethicone/Dimethicone Copolymer, Divinyldimethicone/Dimethicone Crosspolymer, DMAPA Acrylates/Acrylic Acid/Acrylonitrogens Copolymer, Dodecanedioic Acid/Cetearyl Alcohol/Glycol Copolymer, Ethylcellulose, Ethylene/Acrylic Acid Copolymer, Ethylene/Acrylic Acid/VA Copolymer, Ethylene/Calcium Acrylate Copolymer, Ethylene/MA Copolymer, Ethylene/Magnesium Acrylate Copolymer, Ethylene/Methacrylate Copolymer, Ethylene/Octene Copolymer, Ethylene/Propylene Copolymer, Ethylene/Sodium Acrylate Copolymer, Ethylene/VA Copolymer, Ethylene/Zinc Acrylate Copolymer, Ethyl Ester of PVM/MA Copolymer, Euphorbia Cerifera (Candelilla) Wax, Euphorbia Cerifera (Candelilla) Wax Extract, Fibroin/PEG-40/Sodium Acrylate Copolymer, Flexible Collodion, Formaldehyde/Melamine/Tosylamide Copolymer, Galactoarabinan, Glycereth-7 Hydroxystearate/IPDI Copolymer, Glycerin/MA/Rosin Acid Copolymer, Glycerin/Phthalic Acid Copolymer, Glycerin/Phthalic Acid Copolymer Castorate, Glycerin/Succinic Acid Copolymer Castorate, Glyceryl Diricinoleate/IPDI Copolymer, Glyceryl Polyacrylate, Glyceryl Polymethacrylate, Glyceryl Undecyl Dimethicone, Glycidyl C8-11 Acidate/Glycerin/Phthalic Anhydride Copolymer, Glycol Rosinate, Gutta Percha, Hexylene Glycol/Neopentyl Glycol/Adipic Acid/SMDI/DMPA Copolymer, Hydrogenated Brassica Campestris/Aleurites Fordi Oil Copolymer, Hydrogenated Caprylyl Olive Esters, Hydrogenated Cetyl Olive Esters, Hydrogenated Decyl Olive Esters, Hydrogenated Hexyl Olive Esters, Hydrogenated Lauryl Olive Esters, Hydrogenated Myristyl Olive Esters, Hydrogenated Rosin, Hydrogenated Styrene/Butadiene Copolymer, Hydrolyzed Candelilla Wax, Hydrolyzed Carnauba Wax, Hydrolyzed Chitosan, Hydrolyzed Gadidae Protein, Hydrolyzed Jojoba Esters, Hydrolyzed Sunflower Seed Wax, Hydrolyzed Wheat Protein, Hydrolyzed Wheat Protein/Cystine Bis-PG-Propyl Silanetriol Copolymer, Hydrolyzed Wheat Protein/Dimethicone PEG-7 Acetate, Hydrolyzed Wheat Protein/Dimethicone PEG-7 Phosphate Copolymer, Hydrolyzed Wheat Protein/PVP Crosspolymer, Hydroxybutyl Methylcellulose, Hydroxyethylcellulose, Hydroxyethyl Chitosan, Hydroxyethyl Ethylcellulose, Hydroxyethyl/Methoxyethyl Acrylate/Butyl Acrylate Copolymer, Hydroxyethyl/Methoxyethyl Acrylate Copolymer, Hydroxypropylcellulose, Hydroxypropyl Chitosan, Hydroxypropyl Guar, Hydroxypropyl Methylcellulose, Hydroxypropyl Methylcellulose Acetate/Succinate, Hydroxypropyl Oxidized Starch, Hydroxypropyltrimonium Hyaluronate, Hydroxypropyl Xanthan Gum, Isobutylene/Ethylmaleimide/Hydroxyethylmaleimide Copolymer, Isobutylene/MA Copolymer, Isobutylene/Sodium Maleate Copolymer, Isobutylmethacrylate/Bis-Hydroxypropyl Dimethicone Acrylate Copolymer, Isomerized Linoleic Acid, Isophorone Diamine/Cyclohexylamine/Isophthalic Acid/Azelaic Acid Copolymer, Isophoronediamine/Isophthalic Acid/Pentaerythritol Copolymer, Isophorone Diamine/Isophthalic Acid/Trimethylolpropane Copolymer, Isopropyl Ester of PVM/MA Copolymer, 4,4'-Isopropylidenediphenol/Epichlorohydrin Copolymer, Lauryl Acrylate/VA Copolymer, Lauryl Methacrylate/Glycol Dimethacrylate Crosspolymer, Maltodextrin, Mannan, Melia Azadirachta Conditioned Media/Culture, Methacrylic Acid/Sodium Acrylamidomethyl Propane Sulfonate Copolymer, Methacryloyl Ethyl Betaine/Acrylates Copolymer, Methacryloyl Propyltrimethoxysilane, Methoxypolyoxymethylene Melamine, Methyl Ethylcellulose, Methyl Methacrylate/Acrylonitrile Copolymer, Methyl Methacrylate Crosspolymer, Methyl Methacrylate/Glycol Dimethacrylate Crosspolymer, Myrica Cerifera (Bayberry) Fruit Wax, Myroxylon Balsamum (Balsam Tolu) Resin, Myroxylon Pereirae (Balsam Peru) Resin, Nitrocellulose, Nylon-12/6/66 Copolymer, Octadecene/MA Copolymer, Octylacrylamide/Acrylates/Butylaminoethyl Methacrylate Copolymer, Oxymethylene/Melamine Copolymer, Palmitic Acid/Pentaerythritol/Stearic Acid/Terephthalic Acid Copolymer, PEG-150/Decyl Alcohol/SMDI Copolymer, PEG-7 Dimethicone, PEG/PPG-25/25 Dimethicone/Acrylates Copolymer, PEG-150/Stearyl Alcohol/SMDI Copolymer, Pentaerythritol/Terephthalic Acid Copolymer, Pentaerythrityl Cyclohexane Dicarboxylate, Perfluorononylethyl Stearyl Dimethicone, Phenylpropyldimethylsiloxysilicate, Phthalic Acid Denatured With Epoxy Resin Alkyd Resin, Phthalic Anhydride/Adipic Acid/Castor Oil/Neopentyl Glycol/PEG-3/Trimethylolpropane Copolymer, Phthalic Anhydride/Benzoic Acid/Glycerin Copolymer, Phthalic Anhydride/Benzoic Acid/Trimethylolpropane Copolymer, Phthalic Anhydride/Butyl Benzoic Acid/Propylene Glycol Copolymer, Phthalic Anhydride/Glycerin/Glycidyl Decanoate Copolymer, Phthalic Anhydride/Trimellitic Anhydride/Glycols Copolymer, Piperylene/Butene/Pentene Copolymer, Piperylene/Butene/Pentene/Pentadiene Copolymer, Pistacia Lentiscus (Mastic) Gum, Polianthes Tuberosa Extract, Polyacrylamide, Polyacrylamidomethylpropane Sulfonic Acid, Polyacrylate-1, Polyacrylate-2, Polyacrylate-5, Polyacrylate-6, Polyacrylic Acid, Polyamide-1, Polybeta-Alanine, Polybeta-Alanine/Glutaric Acid Crosspolymer, Polybutyl Acrylate, Polybutylene Terephthalate, Polychlorotrifluoroethylene, Polydiethyleneglycol Adipate/IPDI Copolymer, Polydimethylaminoethyl Methacrylate, Polyester-1, Polyester-2, Polyester-3, Polyethylacrylate, Polyethylene, Polyethylene Naphthalate, Polyethylene Terephthalate, Polyethylglutamate, Polyethylmethacrylate, Polyglucuronic Acid, Polyglyceryl-2 Diisostearate/IPDI Copolymer, Polyisobutene, Polylysine, Polymethacrylamide, Polymethacrylamidopropyltrimonium Methosulfate, Polymethacrylic Acid, Polymethyl Acrylate, Polymethylglutamate, Polymethyl Methacrylate, Polyoxyisobutylene/Methylene Urea Copolymer, Polyoxymethylene Melamine, Polypentaerythrityl Terephthalate, Polypentene, Polyperfluoroperhydrophenanthrene, Poly-p-Phenylene Terephthalamide, Polyphosphorylcholine Glycol Acrylate, Polyquaternium-1, Polyquaternium-2, Polyquaternium-4, Polyquaternium-5, Polyquaternium-6, Polyquaternium-7, Polyquaternium-8, Polyquaternium-9, Polyquaternium-10, Polyquaternium-11, Polyquaternium-12, Polyquaternium-13, Polyquaternium-14, Polyquaternium-15, Polyquaternium-16, Polyquaternium-17, Polyquaternium-18, Polyquaternium-19, Polyquaternium-20, Polyquaternium-22, Polyquaternium-24, Polyquaternium-27, Polyquaternium-28, Polyquaternium-29, Polyquaternium-30, Polyquaternium-31, Polyquaternium-32, Polyquaternium-33, Polyquaternium-34, Polyquaternium-35, Polyquaternium-36, Polyquaternium-37, Polyquaternium-39, Polyquaternium-43, Polyquaternium-44, Polyquaternium-45, Polyquaternium-46, Polyquaternium-47, Polyquaternium-48, Polyquaternium-49, Polyquaternium-50, Polyquaternium-51, Polyquaternium-56, Polyquaternium-57, Polyquaternium-61, Polysilicone-6, Polysilicone-8, Polysilicone-11, Polysilicone-14, Polystyrene, Polyurethane-1, Polyurethane-2, Polyurethane-4, Polyurethane-5, Polyurethane-6, Polyurethane-7, Polyurethane-8, Polyurethane-10, Polyurethane-11, Polyurethane-12, Polyurethane-13, Polyvinylacetal Diethylaminoacetate, Polyvinyl Acetate, Polyvinyl Alcohol, Polyvinyl Butyral, Polyvinylcaprolactam, Polyvinyl Chloride, Polyvinyl Imidazolinium Acetate, Polyvinyl Isobutyl Ether, Polyvinyl Laurate, Polyvinyl Methyl Ether, Polyvinyl Stearyl Ether, Potassium Acrylates/Acrylamide Copolymer, Potassium Acrylates/C10-30 Alkyl Acrylate Crosspolymer, Potassium Acrylates/Ethylhexyl Acrylate Copolymer, Potassium Butyl Ester of PVM/MA Copolymer, Potassium Carbomer, Potassium Carrageenan, Potassium Ethyl Ester of PVM/MA Copolymer, PPG-26/HDI Copolymer, PPG-17/IPDI/DMPA Copolymer, PPG-12/SMDI Copolymer, PPG-7/Succinic Acid Copolymer, PPG-26/TDI Copolymer, PPG-10 Tocophereth-30, PPG-20 Tocophereth-50, Propylene Glycol Diricinoleate/IPDI Copolymer, Pseudotsuga Menziesii (Balsam Oregon) Resin, Pullulan, PVM/MA Copolymer, PVM/MA Decadiene Crosspolymer, PVP, PVP Montmorillonite, PVP/VA/Itaconic Acid Copolymer, PVP/VA/Vinyl Propionate Copolymer, Quaternium-22, Rhizobian Gum, Rosin, Rubber Latex, Serum Albumin, Shellac, Sodium Acrylates/Acrolein Copolymer, Sodium Acrylates/Acrylonitrogens Copolymer, Sodium Acrylates/C10-30 Alkyl Acrylates Crosspolymer, Sodium Acrylates Copolymer, Sodium Acrylate/Vinyl Alcohol Copolymer, Sodium Butyl Ester of PVM/MA Copolymer, Sodium Carbomer, Sodium Carboxymethyl Chitin, Sodium Carboxymethyl Starch, Sodium Carrageenan, Sodium C4-12 Olefin/Maleic Acid Copolymer, Sodium DVB/Acrylates Copolymer, Sodium Ethyl Ester of PVM/MA Copolymer, Sodium Isooctylene/MA Copolymer, Sodium MA/Diisobutylene Copolymer, Sodium MA/Vinyl Alcohol Copolymer, Sodium PG-Propyldimethicone Thiosulfate Copolymer, Sodium Polyacrylate, Sodium Polymethacrylate, Sodium Polystyrene Sulfonate, Sodium PVM/MA/Decadiene Crosspolymer, Sodium Styrene/Acrylates Copolymer, Sodium Tauride Acrylates/Acrylic Acid/Acrylonitrogens Copolymer, Starch/Acrylates/Acrylamide Copolymer, Starch Diethylaminoethyl Ether, Stearamidopropyl Dimethicone, Steareth-10 Allyl Ether/Acrylates Copolymer, Stearoyl Epoxy Resin, Stearyl HDI/PEG-50 Copolymer, Stearyl Methacrylate/Perfluorooctylethyl Methacrylate Copolymer, Stearylvinyl Ether/MA Copolymer, Styrax Benzoin Gum, Styrene/Acrylates/Acrylonitrile Copolymer, Styrene/Acrylates/Ammonium Methacrylate Copolymer, Styrene/Acrylates Copolymer, Styrene/Allyl Benzoate Copolymer, Styrene/DVB Crosspolymer, Styrene/Isoprene Copolymer, Styrene/MA Copolymer, Styrene/Methacrylamide/Acrylates Copolymer, Styrene/Methylstyrene/Indene Copolymer, Styrene/VA Copolymer, Styrene/VP Copolymer, Sucrose Benzoate/Sucrose Acetate Isobutyrate/Butyl Benzyl Phthalate Copolymer, Sucrose Benzoate/Sucrose Acetate Isobutyrate/Butyl Benzyl Phthalate/Methyl Methacrylate Copolymer, Sucrose Benzoate/Sucrose Acetate Isobutyrate Copolymer, TEA-Acrylates/Acrylonitrogens Copolymer, TEA-Diricinoleate, TEA-Diricinoleate/IPDI Copolymer, Terephthalic Acid/Isophthalic Acid/Sodium Isophthalic Acid Sulfonate/Glycol Copolymer, Tetradecyloctadecyl Behenate, Tetradecyloctadecyl Myristate, Tetradecyloctadecyl Stearate, Titanium Isostearates, Tosylamide/Epoxy Resin, Tosylamide/Formaldehyde Resin, Tricontanyl PVP, Triethylene Glycol Rosinate, Trimethylol Propane Cyclohexene Dicarboxylate, Trimethylolpropane Triacrylate, Trimethylpentanediol/Isophthalic Acid/Trimellitic Anhydride Copolymer, Trimethylsiloxysilicate/Dimethiconol Crosspolymer, Trimethylsiloxysilylcarbamoyl Pullulan, Triticum Vulgare (Wheat) Protein, Tromethamine Acrylates/Acrylonitrogens Copolymer, VA/Butyl Maleate/Isobornyl Acrylate Copolymer, VA/Crotonates Copolymer, VA/Crotonates/Methacryloxybenzophenone-1 Copolymer, VA/Crotonates/Vinyl Neodecanoate Copolymer, VA/Crotonates/Vinyl Propionate Copolymer, VA/Crotonic Acid/PEG-20M Copolymer, VA/DBM Copolymer, VA/Isobutyl Maleate/Vinyl Neodecanoate Copolymer, VA/Vinyl Butyl Benzoate/Crotonates Copolymer, VA/Vinyl Chloride Copolymer, Vinyl Acetate, Vinylamine/Vinyl Alcohol Copolymer, Vinyl Caprolactam/VP/Dimethylaminoethyl Methacrylate Copolymer, Vinyl Chloride/Vinyl Laurate Copolymer, VP/Dimethiconylacrylate/Polycarbamyl/Polyglycol Ester, VP/Dimethylaminoethylmethacrylate Copolymer, VP/Dimethylaminoethylmethacrylate/Polycarbamyl Polyglycol Ester, VP/Eicosene Copolymer, VP/Hexadecene Copolymer, VP/Polycarbamyl Polyglycol Ester, VP/VA Copolymer, Welan Gum, Yeast Beta-Glucan, Yeast Polysaccharides, Zein.

In einer bevorzugten Ausführungsform enthalten die erfindungsgemäßen Sonnenschutzmittel als Filmbilder mindestens einen untere der INCI-Bezeichnung Carbomer bekannten Filmbildner in den zuvor genannten Mengen.

In einer weiteren bevorzugten Ausführungsform enthalten die erfindungsgemäßen Sonnenschutzmittel als Filmbilder mindestens eine Cellulose und/oder ein zuvor genanntes Cellulosederivat in den zuvor genannten Mengen.

Innerhalb dieser Ausführungsform ist besonders bevorzugt, wenn die erfindungsgemäßen Mittel Hydroxyethylcellulose, Carboxymethylcellulose oder Mischungen davon in einem Gewichtsanteil von 0,1 - 1 Gew.-% am Gesamtgewicht der erfindungsgemäßen Mittel enthalten.

Die erfindungsgemäßen Sonnenschutzmittel können weitere zusätzliche optionale Komponenten enthalten, welche üblicherweise in kosmetischen Haut- und/oder Haarpflegezusammensetzungen verwendet werden und die der behandelten Haut, den Haaren oder den Sonnenschutzmitteln vorteilhafte Eigenschaften verleihen. Bei den zusätzlichen optionalen Bestandteilen ist darauf zu achten, dass ihre Verwendung die Eigenschaften und Wirkungen der erfindungsgemäßen Mittel nicht beeinträchtigt.

Eine erste Gruppe optionaler Komponenten bilden beispielsweise Di-n-alkylether mit insgesamt 12 bis 36 C-Atomen, insbesondere 12 bis 24 C-Atomen, wie etwa Di-n-Octylether, Di-n-Decylether, Din-Nonylether, Di-n-Undecylether, Di-n-Dodecylether, n-Hexylnoctylether, n-Octyl-n-decylether, n-Decyl-n-undecylether, n-Undecyl-n-dodecylether und n-Hexyl-n-undecylether sowie Di-tert-Butylether, Di-Isopentylether, Di-3-Ethyldecylether, tert.-Butyl-n-octylether, Isopentyl-n-octylether und 2-Methylpentyl-n-octylether. Die als Handelsprodukte erhältlichen Verbindungen 1,3-Di-(2-Ethylhexyl)-cyclohexan (Cetiol^{®} S) und Di-n-Octylether (Cetiol^{®} OE) können bevorzugt werden. Sofern vorhanden, können Di-n-alkylether in den erfindungsgemäßen Sonnenschutzmitteln in einem Gewichtsanteil von 0,01 - 2 Gew.-% am Gesamtgewicht der Sonnenschutzmittel enthalten sein.

Die erfindungsgemäßen Sonnenschutzmittel können zusätzlich auch Fettsäurepartialglyceride, also Monoglyceride, Diglyceride und technische Gemische davon enthalten. Bei Verwendung technischer Produkte können produktionsbedingt noch geringe Mengen an Triglyceriden enthalten sein. Geeignete Partialglyceride entsprechen bevorzugt der folgenden Formel,

CH₂O(CH₂CH₂O)ₘR¹ | CHO(CH₂CH₂O)ₙR^{s} | CH₂O(CH₂CH₂O)_{q}R³

wobei R¹, R² und R³ unabhängig voneinander für Wasserstoff oder für eine lineare oder verzweigte, gesättigte und/oder ungesättigte Acylgruppe mit 6 bis 22, bevorzugt 12 bis 18 Kohlenstoffatomen, stehen, unter der Voraussetzung, dass mindestens eine dieser Gruppen für eine Acylgruppe steht und mindestens eine diese Gruppen für Wasserstoff stehen. Die Summe (m+n+q) steht für 0 oder Zahlen von 1 bis 100, bevorzugt für 0 oder 5 bis 25. R¹ steht bevorzugt für eine Acylgruppe und R² und R³ stehen bevorzugt für Wasserstoff und die Summe (m+n+q) ist 0. Typische Beispiele sind Mono- und/oder Diglyceride basierend auf Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie technische Gemische davon. Ölsäuremonoglyceride werden bevorzugt verwendet.

Sofern vorhanden, können Partialglyceride in den erfindungsgemäßen Sonnenschutzmitteln in einem Gewichtsanteil von 0,01 - 2 Gew.-% am Gesamtgewicht der Sonnenschutzmittel enthalten sein.

Als Pflegestoff können die erfindungsgemäßen Sonnenschutzmittel weiterhin mindestens ein Vitamin, ein Provitamin, eine Vitaminvorläufersubstanz und/oder eines der Derivate davon enthalten. Hier werden gemäß der Erfindung Vitamine, Provitamine und Vitaminvorläufersubstanzen bevorzugt, die normalerweise den Gruppen A, B, C, E, F und H zugeordnet sind.

Ähnlich dem Zusatz von Glycerin und/oder Propylenglykol verstärkt der Zusatz von Panthenol die Flexibilität des bei Anwendung des erfindungsgemäßen Mittels ausgebildeten Polymerfilms. Sofern vorhanden, können Vitamine in den erfindungsgemäßen Sonnenschutzmitteln in einem Gewichtsanteil von 0,001 - 0,5 Gew.-% am Gesamtgewicht der Sonnenschutzmittel enthalten sein.

Als Pflegestoff können die erfindungsgemäßen Sonnenschutzmittel ebenso mindestens einen Pflanzenextrakt, aber ebenso Monosaccharide oder Oligosaccharide und/oder Lipide enthalten.

Die hierin offenbarten Sonnenschutzmittel können weiterhin ein Parfüm enthalten. Sofern vorhanden, können Parfums in den erfindungsgemäßen Sonnenschutzmitteln in einem Gewichtsanteil von 0,1 - 1 Gew.-% am Gesamtgewicht der Sonnenschutzmittel enthalten sein.

Weitere optionale Komponenten können ausgewählt sein aus Antioxidantien, Puffern, Farbstoffen, Konservierungsmitteln, pH-Stellmitteln oder Mischungen davon.

Der oder die zusätzlichen optionalen Komponenten können in den erfindungsgemäßen Sonnenschutzmitteln in einem Gewichtsanteil von bis zu 5 Gew.-% am Gesamtgewicht der Mittel enthalten sein.

Die erfindungsgemäße Sonnenschutzzusammensetzung kann durch einen Spender direkt auf die Anwendungsoberfläche aufgetragen werden. Somit wird die Verpackung basierend auf dieser Auftragungsmethode, wie es für den Fachmann gut verständlich ist, angepasst. Der Benutzer verteilt dann die Sonnenschutzzusammensetzung überall dort, wo sie benötigt wird. Der Benutzer kann die Sonnenschutzzusammensetzung auf die Anwendungsoberfläche pumpen, also kann der Spender eine Pumpe einschließen. Der Spender kann ebenso eine Pipette einschließen, um zu ermöglichen, dass die Sonnenschutzzusammensetzung aus dem Spender gezielt in bestimmten Bereichen (wie beispielsweise dem Scheitel) aufgetragen werden kann. Der Spender kann ebenso eine Tröpfcheneinlage einschließen.

Die erfindungsgemäße Sonnenschutzzusammensetzung kann alternativ in der Form eines Sprays auf die Anwendungsoberfläche aufgetragen werden. Das Spray kann ein Pumpspray sein oder das Versprühen kann mit Hilfe von Treibmitteln erreicht werden. Geeignete Treibmittel (Treibgase) sind Propan, Propen, n-Butan, iso-Butan, iso-Buten, n-Pentan, Penten, iso-Pentan, iso-Penten, Methan, Ethan, Dimethylether, Stickstoff, Luft, Sauerstoff, Distickstoffoxid, 1,1,1,3-Tetrafluorethan, Heptafluor-n-propan, Perfluorethan, Monochlordifluormethan, 1,1-Difluorethan, genauer gesagt entweder einzeln oder in Kombination. Hydrophile Treibgase, wie etwa Kohlendioxid, können ebenso vorteilhaft verwendet werden, sofern der Anteil an hydrophilen Gasen gering ausgewählt wird und ein lipophiles Treibgas (zum Beispiel Propan/Butan) im Überschuss vorhanden ist. Propan, n-Butan, iso-Butan und Gemischen dieser Treibgase sind besonders bevorzugt. Es hat sich gezeigt, dass die Verwendung von n-Butan als alleiniges Treibgas besonders bevorzugt sein kann. Gefäße aus Metall (Aluminium, Weißblech, Zinn), geschütztem oder nicht-splitterndem Kunststoff oder Glas, das außen mit Kunststoff beschichtet ist, können als Druckgasbehälter verwendet werden; Druckfestigkeit und Bruchfestigkeit, Korrosionsbeständigkeit, leichte Füllbarkeit, wie ebenso ästhetische Gesichtspunkte, Handlichkeit, Bedruckbarkeit usw. spielen in ihrer Auswahl eine Rolle. Vorzugsweise werden die erfindungsgemäßen Sonnenschutzmittel als Pumpspray konfektioniert, denn dadurch wird gewährleistet, dass mit nur einem Mittel in einem einzigen Behandlungsschritt alle Bereiche des Kopfes (Gesicht, Hals, Haare) vor Sonneneinstrahlung geschützt und gepflegt werden können.

Die Erfindung betrifft ebenso die Verwendung des Sonnenschutzmittels für den Schutz der Haut, insbesondere der Kopfhaut und der Haare, vor UV-Strahlen sowie zur Pflege von Haut und Haaren.

Ein weiterer Gegenstand der vorliegenden Anmeldung ist ein Verfahren zum Verwenden des erfindungsgemäßen Sonnenschutzmittels, wobei das Verfahren das Aufbringen des Sonnenschutzmittels auf die Haare und/oder die Haut, insbesondere die Gesichts- oder Kopfhaut, umfasst.

Die Erfindung kann durch die folgenden nummerierten Aussagen gekennzeichnet sein.
1. Kosmetisches Sonnenschutzmittel, enthaltend - bezogen auf das Gesamtgewicht des Mittels -
   a) 10 - 30 Gew.-% einer UV-Filtermischung, umfassend mindestens einen UVA- und mindestens einen UVB-Filter,
   b) 1 - 10 Gew.-% mindestens eines Öls mit einem Spreitwert > 500 mm²/10 min. und
   c) 50 - 80 Gew.-% mindestens eines C1-C6-Alkohols.
2. Kosmetisches Mittel nach Aussage 1, enthaltend als UVA-Filter Bemotrizinol, Avobenzone, (Butyl Methoxydibenzoylmethane) oder Mischungen davon.
3. Kosmetisches Mittel nach einer der vorhergehenden Aussagen, enthaltend Avobenzone in einem Gewichtsanteil von 1 bis 10 Gew.-% am Gesamtgewicht des Sonnenschutzmittels.
4. Kosmetisches Mittel nach einer der vorhergehenden Aussagen, enthaltend als UVB-Filter Octisalate (Ethylhexyl Salicylate), Homosalate, Octocrylene, Ensulizol (Phenylbenzimidazole sulfonic acid) oder Mischungen davon.
5. Kosmetisches Mittel nach einer der vorhergehenden Aussagen, enthaltend Homosalate, Octocrylene oder Mischungen davon in einem Gewichtsanteil von 10 bis 30 Gew.-% am Gesamtgewicht des Sonnenschutzmittels.
6. Kosmetisches Mittel nach einer der vorhergehenden Aussagen, enthaltend - bezogen auf das Gesamtgewicht des Mittels -
   (i) 1 - 10 Gew.-% Avobenzone,
   (ii) 5 - 15 Gew.-% Homosalate und
   (iii) 5 - 15 Gew.-% Octocrylene.
7. Kosmetisches Mittel nach einer der Aussagen 1 bis 4, enthaltend Ethylhexyl Salicylate, Octocrylene, Phenylbenzimidazole sulfonic acid oder Mischungen davon in einem Gewichtsanteil von 10 bis 30 Gew.-% am Gesamtgewicht des Sonnenschutzmittels.
8. Kosmetisches Mittel nach Aussage 7, enthaltend - bezogen auf das Gesamtgewicht des Mittels
   (i) 1 - 10 Gew.-% Ethylhexyl Salicylate,
   (ii) 5 - 15 Gew.-% Octocylene und
   (iii) 1 - 10 Gew.-% Phenylbenzimidazole sulfonic acid.
9. Kosmetisches Mittel nach einer der vorhergehenden Aussagen, enthaltend mindestens eines der unter den INCI-Bezeichnungen Isopropyl palmitate, Isopropyl myristate, Coco caprylate/caprate, Isopropyl isostearate, Butylene Glycol dicaprylate/dicaprate, Di-(2-ethylhexyl) carbonate, Dicaprylyl carbonate, Isopropyl stearate, Ethylhexyl isononanoate, Ethylhexyl cocoate, Dibutyl adipate, Isodecyl neopentanoate, Isodecane, Isoundecane, Isododecane, Isotridecane, Isotertadecane, Isopentadecane, Isohexadecane, Dicaprylyl Ether, Hexyl Laurate, Heptyl Undecylate bekannten Öle oder natürliche Öle wie beispielsweise Squalan, Babassuöl, Kokosöl oder beliebige Mischungen dieser Öle.
10. Kosmetisches Mittel nach Aussage 9, enthaltend Dicaprylyl carbonate in einem Gewichtsanteil von 2 - 7,5 Gew.-% am Gesamtgewicht des Mittels.
11. Kosmetisches Mittel nach Aussage 9, enthaltend Dicaprylyl carbonate in einem Gewichtsanteil von 2 - 7,5 Gew.-% und 0,05 - 1 Gew.-% Kokosöl am Gesamtgewicht des kosmetischen Mittels.
12. Kosmetisches Mittel nach Aussage 9, enthaltend Isopropylmyristat, Ethylhexyl isononanoate oder Mischungen davon in einem Gewichtsanteil von 2 - 9 Gew.-% am Gesamtgewicht des Mittels.
13. Kosmetisches Mittel nach Aussage 9, enthaltend Isopropylmyristat, Ethylhexyl isononanoate oder Mischungen davon in einem Gewichtsanteil von 2 - 9 Gew.-% und Kokosöl in einem Gewichtsanteil von 0,05 - 1 Gew.-% am Gesamtgewicht des kosmetischen Mittels.
14. Kosmetisches Mittel nach einer der vorhergehenden Aussagen, enthaltend Ethanol in einem Gewichtsanteil von 55 bis 80 Gew.-% am Gesamtgewicht des kosmetischen Mittels.
15. Kosmetisches Mittel nach einer der vorhergehenden Aussagen, enthaltend Ethanol in einem Gewichtsanteil von 55 bis 80 Gew.-% sowie 1,2-Propylenglycol, Glycerin oder Mischungen davon in einem Gewichtsanteil von 0 - 10 Gew.-% am Gesamtgewicht des kosmetischen Mittels.
16. Kosmetisches Mittel nach einer der vorhergehenden Aussagen, welches frei von Silikonen ist.
17. Kosmetisches Mittel nach einer der vorhergehenden Aussagen, welches frei von zugesetztem Wasser ist.
18. Kosmetisches Mittel nach einer der vorhergehenden Aussagen, enthaltend mindestens einen Filmbildner in einem Gewichtsanteil von 0,1 bis 1,5 Gew.-% am Gesamtgewicht des Mittels.
19. Kosmetisches Mittel nach Aussage 18, umfassend unter der INCI-Bezeichnung Carbomer bekannte Filmbildner.
20. Kosmetisches Mittel nach Aussage 18, umfassend Cellulose und/oder deren Derivate.
21. Kosmetisches Mittel nach einer der vorhergehenden Aussagen, enthaltend mindestens ein Vitamin, ein Provitamin, eine Vitaminvorläufersubstanz und/oder eines der Derivate davon in einem Gewichtsanteil von 0,001 - 0,5 Gew.-% am Gesamtgewicht des Mittels.
22. Kosmetisches Mittel nach einer der vorhergehenden Aussagen, enthaltend Panthenol in einem Gewichtsanteil von 0,001 - 0,5 Gew.-% am Gesamtgewicht des Mittels.
23. Kosmetisches Mittel nach einer der vorhergehenden Aussagen in Form eines Serums.
24. Kosmetisches Mittel nach einer der vorhergehenden Aussagen für die Behandlung des Kopfes.
25. Kosmetisches Mittel nach einer der vorhergehenden Aussagen für die Behandlung der Haare, der Kopfhaut, des Gesichts und des Halses.
26. Kosmetisches Produkt, umfassend ein kosmetisches Mittel nach einer der vorhergehenden Aussagen.
27. Kosmetisches Produkt nach Aussage 26, das einen Pumpspender umfasst.
28. Kosmetisches Produkt nach Aussage 26, das eine Pipette umfasst.
29. Kosmetisches Produkt nach Aussage 26, das eine Tröpfcheneinlage umfasst.
30. Verwendung eines kosmetischen Mittels oder eines kosmetischen Produkts nach einer der vorhergehenden Aussagen für den Schutz der Haut, insbesondere der Kopfhaut und der Haare, vor UV-Strahlen sowie zur Pflege von Haut und Haaren.
31. Verfahren zum Verwenden eines kosmetischen Mittels nach einer der Aussagen 1 bis 25 oder eines Produkts nach einem der Ansprüche 26 bis 30, wobei das Verfahren das Aufbringen eines kosmetischen Mittels auf die Haare und/oder die Haut, insbesondere die Gesichts- oder Kopfhaut, umfasst.

### Ausführungsbeispiele:

Die folgenden Beispiele sollen den Gegenstand der vorliegenden Erfindung erläutern, ohne ihn jedoch zu beschränken (Angaben in Gew.-% bezogen auf das Gesamtgewicht des Sonnenschutzmittels, sofern nicht anders angegeben).

| | Formel 1 | Formel 2 | Formel 3 | Formel 4 |
|---|---|---|---|---|
| UVA-Filter + UVB-Filter | 10-30 | 12,5 - 30 | 15-30 | 17,5-27,5 |
| Öl* | 1 - 10 | 2,5 - 10 | 3-9 | 4-8 |
| C1-C6-Alkohol | 50 - 80 | 55 - 80 | 60 - 80 | 65 - 80 |
| ggfs. weitere Hilfs- und Zusatzstoffe | ad 100 | ad 100 | ad 100 | ad 100 |

| | | | | |
|---|---|---|---|---|
| * mit Spreitwert > 500 mm²/10 min. | | | | |

| | Formel 5 | Formel 6 | Formel 7 | Formel 8 |
|---|---|---|---|---|
| Avobenzone und/oder Bemotrizinol | 1 - 10 | 2-9 | 2,5 - 8 | 3 - 7,5 |
| Octisalate, Homosalate, Octocrylene und/oder Ensulizol | 10-30 | 12-30 | 12,5 - 27,5 | 12,5 - 25 |
| Öl* | 1 - 10 | 2,5 - 10 | 3-9 | 4-8 |
| C1-C6-Alkohol | 50 - 80 | 55 - 80 | 60 - 80 | 65 - 80 |
| ggfs. weitere Hilfs- und Zusatzstoffe | ad 100 | ad 100 | ad 100 | ad 100 |

| | | | | |
|---|---|---|---|---|
| * mit Spreitwert > 500 mm²/10 min. | | | | |

| | Formel 9 | Formel 10 | Formel 11 | Formel 12 |
|---|---|---|---|---|
| UVA-Filter + UVB-Filter | 10-30 | 12,5 - 30 | 15-30 | 17,5-27,5 |
| Öl* | 1 - 10 | 2,5 - 10 | 3-9 | 4-8 |
| C1-C6-Alkohol | 50 - 80 | 55 - 80 | 60 - 80 | 65 - 80 |
| ggfs. weitere Hilfs- und Zusatzstoffe | ad 100 | ad 100 | ad 100 | ad 100 |

| | | | | |
|---|---|---|---|---|
| * Isopropyl palmitate, Isopropyl myristate, Coco caprylate/caprate, Isopropyl isostearate, Butylene Glycol dicaprylate/dicaprate, Di-(2-ethylhexyl) carbonate, Dicaprylyl carbonate, Isopropyl stearate, Ethylhexyl isononanoate, Ethylhexyl cocoate, Dibutyl adipate, Isodecyl neopentanoate, Isodecane, Isoundecane, Isododecane, Isotridecane, Isotertadecane, Isopentadecane, Isohexadecane, Dicaprylyl Ether, Hexyl Laurate, Heptyl Undecylate und/oder Squalan, Babassuöl, Kokosöl | | | | |

| | Formel 13 | Formel 14 | Formel 15 | Formel 16 |
|---|---|---|---|---|
| UVA-Filter + UVB-Filter | 10-30 | 12,5 - 30 | 15-30 | 17,5-27,5 |
| Dicaprylyl carbonate und/oder Isododecane | 1 - 10 | 1,5-9 | 1,5-8 | 2 - 7,5 |
| C1-C6-Alkohol | 50 - 80 | 55 - 80 | 60 - 80 | 65 - 80 |
| ggfs. weitere Hilfs- und Zusatzstoffe | ad 100 | ad 100 | ad 100 | ad 100 |

| | Formel 17 | Formel 18 | Formel 19 | Formel 20 |
|---|---|---|---|---|
| UVA-Filter + UVB-Filter | 10-30 | 12,5 - 30 | 15-30 | 17,5-27,5 |
| Isopropylmyristat und/oder Ethylhexyl isononanoate | 1 - 10 | 1,5-9,5 | 1,5-9 | 2-9 |
| C1-C6-Alkohol | 50 - 80 | 55 - 80 | 60 - 80 | 65 - 80 |
| ggfs. weitere Hilfs- und Zusatzstoffe | ad 100 | ad 100 | ad 100 | ad 100 |

| | Formel 21 | Formel 22 | Formel 23 | Formel 24 |
|---|---|---|---|---|
| UVA-Filter + UVB-Filter | 10-30 | 12,5 - 30 | 15-30 | 17,5-27,5 |
| Öl* | 1 - 10 | 2,5 - 10 | 3-9 | 4-8 |
| Ethanol | 50 - 80 | 55 - 80 | 60 - 80 | 65-75 |
| 1,2-Propylenglykol und/oder Glycerin | 0-10 | 0,1 - 9 | 0,25 - 8 | 0,5 - 7,5 |
| ggfs. weitere Hilfs- und Zusatzstoffe | ad 100 | ad 100 | ad 100 | ad 100 |

| | | | | |
|---|---|---|---|---|
| * mit Spreitwert > 500 mm²/10 min. | | | | |

| | Formel 25 | Formel 26 | Formel 27 | Formel 28 |
|---|---|---|---|---|
| UVA-Filter + UVB-Filter | 10-30 | 12,5 - 30 | 15-30 | 17,5-27,5 |
| Öl* | 1 - 10 | 2,5 - 10 | 3-9 | 4-8 |
| C1-C6-Alkohol | 50 - 80 | 55 - 80 | 60 - 80 | 65 - 80 |
| Filmbildner | 0,1 - 1,5 | 0,2 - 1,5 | 0,25 - 1,25 | 0,25 - 1 |
| ggfs. weitere Hilfs- und Zusatzstoffe | ad 100 | ad 100 | ad 100 | ad 100 |

| | | | | |
|---|---|---|---|---|
| * mit Spreitwert > 500 mm²/10 min. | | | | |

| | Formel 29 | Formel 30 | Formel 31 | Formel 32 |
|---|---|---|---|---|
| Avobenzone | 1 - 10 | 2-9 | 2,5 - 8 | 3 - 7,5 |
| Homosalate und/oder Octocrylene | 10-30 | 12-30 | 12,5 - 27,5 | 12,5 - 25 |
| Dicaprylyl carbonate und/oder Isododecane | 1 - 10 | 1,5-9 | 1,5-8 | 2 - 7,5 |
| Kokosöl | 0,05 - 1 | 0,05 - 0,9 | 0,1 - 0,8 | 0,15 - 0,75 |
| C1-C6-Alkohol | 50 - 80 | 55 - 80 | 60 - 80 | 65 - 80 |
| ggfs. weitere Hilfs- und Zusatzstoffe | ad 100 | ad 100 | ad 100 | ad 100 |

| | Formel 33 | Formel 34 | Formel 35 | Formel 36 |
|---|---|---|---|---|
| Avobenzone | 1 - 10 | 2-9 | 2,5 - 8 | 3 - 7,5 |
| Homosalate und/oder Octocrylene | 10-30 | 12-30 | 12,5 - 27,5 | 12,5 - 25 |
| Dicaprylyl carbonate und/oder Isododecane | 1 - 10 | 1,5-9 | 1,5-8 | 2 - 7,5 |
| Kokosöl | 0,05 - 1 | 0,05 - 0,9 | 0,1 - 0,8 | 0,15 - 0,75 |
| Ethanol | 50 - 80 | 55 - 80 | 60 - 80 | 65-75 |
| 1,2-Propylenglykol und/oder Glycerin | 0-10 | 0,1 - 9 | 0,25 - 8 | 0,5 - 7,5 |
| ggfs. weitere Hilfs- und Zusatzstoffe | ad 100 | ad 100 | ad 100 | ad 100 |

| | Formel 37 | Formel 38 | Formel 39 | Formel 40 |
|---|---|---|---|---|
| Avobenzone | 1 - 10 | 2-9 | 2,5 - 8 | 3 - 7,5 |
| Homosalate und/oder Octocrylene | 10-30 | 12-30 | 12,5 - 27,5 | 12,5 - 25 |
| Dicaprylyl carbonate und/oder Isododecane | 1 - 10 | 1,5-9 | 1,5-8 | 2 - 7,5 |
| Kokosöl | 0,05 - 1 | 0,05 - 0,9 | 0,1 - 0,8 | 0,15 - 0,75 |
| Ethanol | 50 - 80 | 55 - 80 | 60 - 80 | 65-75 |
| 1,2-Propylenglykol und/oder Glycerin | 0-10 | 0,1 - 9 | 0,25 - 8 | 0,5 - 7,5 |
| Filmbildner* | 0,1 - 1,5 | 0,2 - 1,5 | 0,25 - 1,25 | 0,25 - 1 |
| ggfs. weitere Hilfs- und Zusatzstoffe | ad 100 | ad 100 | ad 100 | ad 100 |

| | | | | |
|---|---|---|---|---|
| * Carbomer, Cellulose, Hydroxyethylcellulose, Carboxymethylcellulose | | | | |

| | Formel 41 | Formel 42 | Formel 43 | Formel 44 |
|---|---|---|---|---|
| Avobenzone | 1 - 10 | 2-9 | 2,5 - 8 | 3 - 7,5 |
| Octisalate, Homosalate, Octocrylene und/oder Ensulizol | 10-30 | 12-30 | 12,5 - 27,5 | 12,5 - 25 |
| Isopropylmyristat und/oder Ethylhexyl isononanoate | 1 - 10 | 1,5-9,5 | 1,5-9 | 2-9 |
| Kokosöl | 0-1 | 0,05 - 0,9 | 0,1 - 0,8 | 0,15 - 0,75 |
| C1-C6-Alkohol | 50 - 80 | 55 - 80 | 60 - 80 | 65 - 80 |
| ggfs. weitere Hilfs- und Zusatzstoffe | ad 100 | ad 100 | ad 100 | ad 100 |

| | Formel 45 | Formel 46 | Formel 47 | Formel 48 |
|---|---|---|---|---|
| Avobenzone | 1 - 10 | 2-9 | 2,5 - 8 | 3 - 7,5 |
| Octisalate, Homosalate, Octocrylene und/oder Ensulizol | 10-30 | 12-30 | 12,5 - 27,5 | 12,5 - 25 |
| Isopropylmyristat und/oder Ethylhexyl isononanoate | 1 - 10 | 1,5-9,5 | 1,5-9 | 2-9 |
| Kokosöl | 0-1 | 0,05 - 0,9 | 0,1 - 0,8 | 0,15 - 0,75 |
| Ethanol | 50 - 80 | 55 - 80 | 60 - 80 | 65-75 |
| 1,2-Propylenglykol und/oder Glycerin | 0-10 | 0,1 - 9 | 0,25 - 8 | 0,5 - 7,5 |
| ggfs. weitere Hilfs- und Zusatzstoffe | ad 100 | ad 100 | ad 100 | ad 100 |

| | Formel 49 | Formel 50 | Formel 51 | Formel 52 |
|---|---|---|---|---|
| Avobenzone | 1 - 10 | 2-9 | 2,5 - 8 | 3 - 7,5 |
| Octisalate, Homosalate, Octocrylene und/oder Ensulizol | 10-30 | 12-30 | 12,5 - 27,5 | 12,5 - 25 |
| Isopropylmyristat und/oder Ethylhexyl isononanoate | 1 - 10 | 1,5-9,5 | 1,5-9 | 2-9 |
| Kokosöl | 0-1 | 0,05 - 0,9 | 0,1 - 0,8 | 0,15 - 0,75 |
| Ethanol | 50 - 80 | 55 - 80 | 60 - 80 | 65-75 |
| 1,2-Propylenglykol und/oder Glycerin | 0-10 | 0,1 - 9 | 0,25 - 8 | 0,5 - 7,5 |
| Filmbildner* | 0-1,5 | 0,1 - 1,5 | 0,1 - 1,25 | 0,1 - 1 |
| ggfs. weitere Hilfs- und Zusatzstoffe | ad 100 | ad 100 | ad 100 | ad 100 |

| | | | | |
|---|---|---|---|---|
| * Carbomer, Cellulose, Hydroxyethylcellulose und/oder Carboxymethylcellulose | | | | |

Die erfindungsgemäßen Sonnenschutzzusammensetzungen wurden in einen Pumpspender abgefüllt und gleichmäßig auf Haaren, Gesicht und Hals verteilt.

## Patentansprüche

1. Kosmetisches Sonnenschutzmittel, enthaltend - bezogen auf das Gesamtgewicht des Mittels -
a) 10 - 30 Gew.-% einer UV-Filtermischung, umfassend mindestens einen UVA- und mindestens einen UVB-Filter,
b) 1 - 10 Gew.-% mindestens eines Öls mit einem Spreitwert > 500 mm²/10 min. und
c) 50 - 80 Gew.-% mindestens eines C1-C6-Alkohols.

2. Kosmetisches Mittel nach Anspruch 1, enthaltend
- als UVA-Filter Bemotrizinol, Avobenzone, (Butyl Methoxydibenzoylmethane) oder Mischungen davon
- als UVB-Filter Octisalate (Ethylhexyl Salicylate), Homosalate, Octocrylene, Ensulizol (Phenylbenzimidazole sulfonic acid) oder Mischungen davon.

3. Kosmetisches Mittel nach einem der vorhergehenden Ansprüche, enthaltend - bezogen auf das Gesamtgewicht des Mittels -
(i) 1 - 10 Gew.-% Avobenzone,
(ii) 5 - 15 Gew.-% Homosalate,
(iii) 5 - 15 Gew.-% Octocrylene.

4. Kosmetisches Mittel nach einem der Ansprüche 1 bis 3, enthaltend - bezogen auf das Gesamtgewicht des Mittels -
(i) 1 - 10 Gew.-% Ethylhexyl Salicylate,
(ii) 5 - 15 Gew.-% Octocylene und
(iii) 1 - 10 Gew.-% Phenylbenzimidazole sulfonic acid.

5. Kosmetisches Mittel nach einem der vorhergehenden Ansprüche, enthaltend mindestens eines der unter den INCI-Bezeichnungen Isopropyl palmitate, Isopropyl myristate, Coco caprylate/caprate, Isopropyl isostearate, Butylene Glycol dicaprylate/dicaprate, Di-(2-ethylhexyl) carbonate, Dicaprylyl carbonate, Isopropyl stearate, Ethylhexyl isononanoate, Ethylhexyl cocoate, Dibutyl adipate, Isodecyl neopentanoate, Isodecane, Isoundecane, Isododecane, Isotridecane, Isotertadecane, Isopentadecane, Isohexadecane, Dicaprylyl Ether, Hexyl Laurate, Heptyl Undecylate bekannten Öle oder natürliche Öle, wie beispielsweise Squalan, Babassuöl, Kokosöl oder beliebige Mischungen dieser Öle.

6. Kosmetisches Mittel nach Anspruch 5, enthaltend Dicaprylyl carbonate in einem Gewichtsanteil von 2 - 7,5 Gew.-% am Gesamtgewicht des kosmetischen Mittels.

7. Kosmetisches Mittel nach Anspruch 5, enthaltend Isopropylmyristat, Ethylhexyl isononanoate oder Mischungen davon in einem Gewichtsanteil von 2 - 9 Gew.-% am Gesamtgewicht des Mittels.

8. Kosmetisches Mittel nach einem der vorhergehenden Ansprüche, enthaltend Ethanol in einem Gewichtsanteil von 55 bis 80 Gew.-% sowie 1,2-Propylenglycol, Glycerin oder Mischungen davon in einem Gewichtsanteil von 0 - 10 Gew.-% am Gesamtgewicht des kosmetischen Mittels.

9. Kosmetisches Mittel nach einem der vorhergehenden Ansprüche, enthaltend mindestens einen Filmbildner in einem Gewichtsanteil von 0,1 bis 1,5 Gew.-% am Gesamtgewicht des kosmetischen Mittels.

10. Kosmetisches Mittel nach Anspruch 9, enthaltend als Filmbildner unter der INCI-Bezeichnung Carbomer bekannte Verbindungen, Cellulose und/oder Cellulosederivate.

11. Kosmetisches Mittel nach einem der Ansprüche 9 oder 10, enthaltend Cellulose oder ein Cellulosederivat ausgewählt aus Hydroxyethylcellulose, Carboxymethylcellulose oder Mischungen davon.

12. Kosmetisches Mittel nach einem der vorhergehenden Ansprüche in Form eines Serums.

13. Kosmetisches Produkt nach einem der vorhergehenden Ansprüche, das einen Pumpspender umfasst.

14. Verwendung eines kosmetischen Mittels nach einem der Ansprüche 1 bis 12 oder eines kosmetischen Produkts nach Anspruch 13 für den Schutz der Haut, insbesondere der Kopfhaut und der Haare, vor UV-Strahlen sowie zur Pflege von Haut und Haaren.

15. Verfahren zum Verwenden eines kosmetischen Mittels nach einem der Ansprüche 1 bis 12, wobei das Verfahren das Aufbringen eines kosmetischen Mittels auf die Haare und/oder die Haut, insbesondere die Gesichts- oder Kopfhaut, umfasst.
